# FASCICULE DE BREVET EUROPEEN

(11) **EP 1 046 389 B1**
(45) Date de publication et mention de la délivrance du brevet: **13.08.2003**
(21) Numéro de dépôt: 00401101.1
(22) Date de dépôt: 20.04.2000
(51) Int. Cl.: A61K 7/021, A61K 7/48

(54) **Composition cosmétique, notamment de maquillage, contenant un pigment dérivé de pyrrolopyrrole**
Kosmetische Zusammensetzung insbesondere eine make-up Zusammensetzung die ein Pyrrolopyrrolpigment enthält
Cosnetic composition in particular a make-up composition comprising a pyrrolopyrrole pigment

(30) Priorité: 22.04.1999 FR 9905134
(43) Date de publication de la demande: 25.10.2000
(73) Titulaire: L'OREAL, 75008 Paris (FR)
(72) Inventeur: Simon, Jean-Christophe, 75012 Paris (FR)
(74) Mandataire: Dossmann, Gérard

(56) Documents cités:
- EP-A- 0 133 156
- EP-A- 0 787 731
- EP-A- 0 811 625
- EP-A- 0 849 221
- US-A- 5 786 487

## Description

La présente invention se rapporte à des compositions cosmétiques contenant un nouveau pigment orangé de couleur intense et saturée, non générateur de radicaux libres et plus spécialement à des compositions de maquillage de la peau aussi bien du visage que du corps humain, des fibres kératiniques telles que les cils, les sourcils ou les cheveux, et des lèvres.

Les compositions de maquillage telles que les poudres libres ou compactées, les fonds de teint, les fards à joues ou à paupières, les rouges à lèvres, les anti-cernes, les mascaras, les eye-liners, les crayons à lèvres ou à yeux ou encore les produits de maquillage du corps, sont constituées d'un véhicule approprié et d'agents de coloration de natures différentes, destinés à conférer une certaine couleur à ces compositions, avant et/ou après leur application sur la peau, les lèvres et/ou les fibres kératiniques.

Ces agents de coloration peuvent être des laques, des pigments minéraux ou organiques et/ou des pigments nacrés ou encore des colorants. Dans la gamme des pigments orangés, les cosméticiens disposent de pigments d'origine minérale comme les oxydes de fer ou les mélanges d'oxydes de fer brun-jaune et de pigments d'origine organique. Les pigments minéraux, et en particulier les oxydes minéraux, ont l'avantage d'être très stables mais ont l'inconvénient de donner des couleurs plutôt ternes et pâles. Les laques organiques ont l'avantage de conférer aux compositions des couleurs vives, mais sont pour la plupart instables à la lumière, à la température ou au pH. Certaines de ces laques présentent également l'inconvénient de tacher la peau de manière disgracieuse après application, par dégorgement du colorant. Les pigments nacrés quant à eux permettent d'obtenir des couleurs variées, mais jamais intenses, à effets irisés mais le plus souvent assez faibles.

Par ailleurs, certains agents de coloration présentent l'inconvénient d'engendrer des radicaux libres dans les formules de maquillage modifiant le rendu des couleurs et la stabilité des compositions, puis sur la peau après application, ce qui favorise le vieillissement cutané (apparition de rides, ridules, jaunissement de la peau). Comme agents de coloration présentant cet inconvénient, on peut citer notamment les mélanges d'oxydes de fer brun-jaune vendus sous la dénomination commerciale "SICOMET BRUN ZP 3569" par BASF par exemple, les pigments d'origine organique comme le pigment Orange 5 (CI 12075) ou D&C Orange N° 5 (CI 45730 : 1) ou D&C Orange N° 4 (CI 15510), ainsi que les laques d'aluminium D&C Orange N° 5 Aluminum Lake, D&C Orange N° 4 Aluminium Lake ou D & C Orange N° 10 Aluminium Lake.

Aujourd'hui, pour pallier cet inconvénient, on utilise des agents anti-oxydants comme par exemple l'éthoxyquine. Malheureusement, il est souvent difficile de trouver un agent anti-oxydant efficace à 100% compte tenu de la multiplicité des ingrédients présents dans les compositions de maquillage. De plus, les agents anti-oxydants engendrent souvent eux-mêmes par oxydation des produits de dégradation qui peuvent être gênants.

La présente invention a pour objet l'utilisation dans une composition cosmétique d'un nouveau pigment orangé de couleur intense et saturée, stable et ayant l'avantage d'engendrer beaucoup moins de radicaux libres que les pigments classiquement utilisés, notamment pour obtenir une couleur orangée.

De façon surprenante, la demanderesse a trouvé que certains dérivés de dicétodiarylpyrrolopyrroles (DPP en abrégé) permettaient de limiter la production de radicaux libres, puisqu'ils ont la propriété d'engendrer très peu de radicaux libres, et d'éviter ainsi l'utilisation d'anti-oxydants dans les compositions. En outre, ces pigments permettent d'obtenir une coloration orangée intense, très vive, non dégorgeante sur la peau, stable à la lumière, au pH et à la température.

De façon plus précise, l'invention a pour objet une composition cosmétique colorée pour application topique et plus spécialement une composition cosmétique de maquillage contenant, dans un milieu cosmétiquement acceptable une phase grasse et un pigment dérivé de dicétodiarylpyrrolopyrrole de formule (I) : 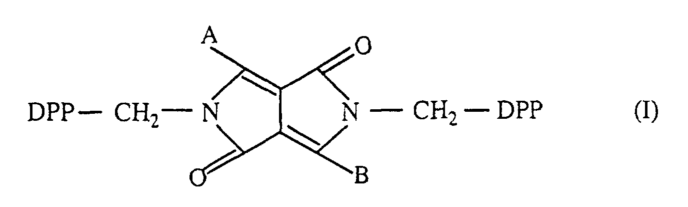 dans laquelle A et B sont des radicaux aryle identiques ou différents et DPP est un radical dicétodiarylpyrrolopyrrolidyle, le dérivé de dicétodiarylpyrrolopyrrole de formule (I) étant substitué par 0 à 6 moles de -SO₃M par mole de dérivé de pyrrolopyrrole, M étant un atome d'hydrogène ou un cation métallique ou ammonium.

Lorsque M est un cation métallique, il est de préférence un cation sodium, potassium ou lithium.

De préférence, le dérivé de pyrrolopyrrole de formule (I) est substitué par 0 à 2 moles de -SO₃M, et plus préférentiellement par 0 à 0,75 mole de -SO₃M par mole de dérivé de pyrrolopyrrole de formule (I).

Des dérivés préférés de pyrrolopyrrole ne contiennnent pas de radical -SO₃M.

De préférence, DPP est un radical 1,4-dicéto-3,6-diarylpyrrolo-[3,4-c]pyrrolidyle de formule : 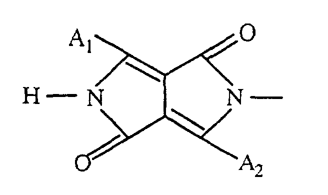 dans laquelle A₁ et A₂ sont des radicaux aryle identiques ou différents.

A, B, A₁ et A₂ peuvent être des radicaux aromatiques ou hétéroaromatiques.

Des radicaux particulièrement appropriés sont ceux de formules : 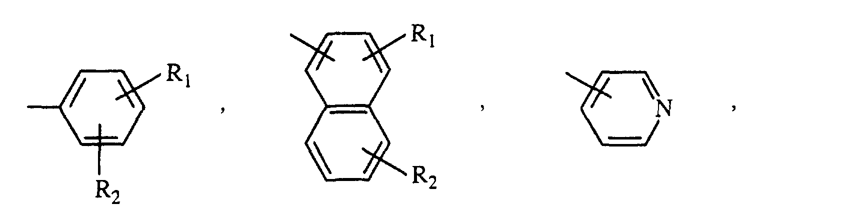 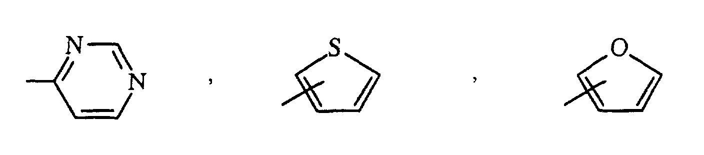 ou 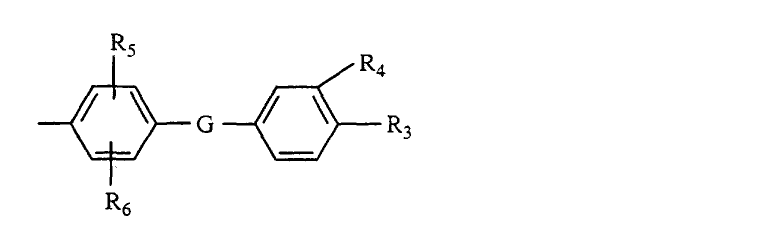 dans lesquelles :
R₁ et R₂ désignent indépendamment l'un de l'autre un atome d'hydrogène ou d'halogène, un radical C₁-C₁₈ alkyle, C₁-C₁₈ alcoxy, C₁-C₁₈ alkylmercapto, C₁-C₁₈ alkylamino, C₁-C₁₈ alcoxycarbonyle, C₁-C₁₈ alkylaminocarbonyle, -CN, -NO₂, trifluorométhyle, C₅-C₆ cycloalkyle, -CH=N-(C₁-C₁₈ alkyle), phényle, 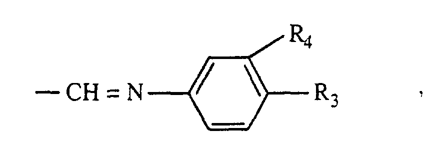 imidazolyle, pyrazolyle, triazolyle, pipérazinyle, pyrrolyle, oxazolyle, benzoxazolyle, benzothiazolyle, benzimidazolyle, morpholinyle, pipéridinyle ou pyrrolidinyle,
G désigne -CH₂-, -CH(CH₃)-, -C(CH₃)₂-, -CH=N-, -N=N-, -O-, -S-, -SO-, -SO₂-, -CONH- ou -NR₇-,
R₃ et R₄ désignent, indépendamment l'un de l'autre, un atome d'hydrogène ou d'halogène, un radical C₁-C₆ alkyle, C₁-C₁₈ alcoxy ou -CN, R₅ et R₆ désignent, indépendamment l'un de l'autre, un atome d'hydrogène ou d'halogène ou un radical C₁-C₆ alkyle, et
R₇ est un atome d'hydrogène ou un radical C₁-C₆ alkyle.

En particulier, A, B, A₁ et A₂ désignent chacun un groupe de formule : 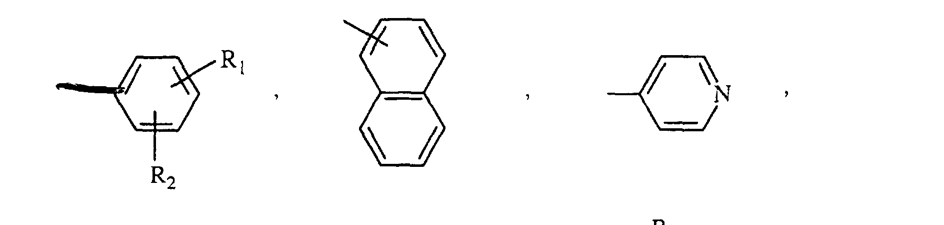 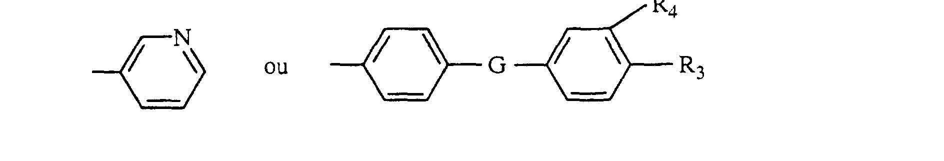 dans laquelle :
R₁ et R₂ désignent, indépendamment l'un de l'autre, un atome d'hydrogène, de chlore ou de brome, un radical C₁-C₄ alkyle, C₁-C₆ alcoxy, C₁-C₆ alkylamino, phényle ou -CN,
G désigne -O-, -NR₇-, -N=N- ou -SO₂-,
R₃ et R₄ désignent de l'hydrogène, et
R₇ est un atome d'hydrogène, un radical méthyle ou éthyle.

Plus particulièrement, A, B, A₁ et A₂ désignent chacun un groupe de formule : 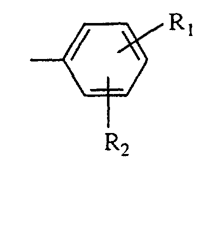 dans laquelle : R₁ et R₂ désignent chacun, indépendamment l'un de l'autre, un atome d'hydrogène, de chlore ou de brome, un radical méthyle, tert.-butyle, phényle ou -CN.

Au moins l'un des radicaux R₁ et R₂ est de préférence un atome d'hydrogène.

Plus préférentiellement, au moins l'un des radicaux R₁ et R₂ est un atome d'hydrogène et l'autre est en position 3- ou 4- du noyau phényle.

Des composés particulièrement préférés sont ceux dans lesquels A et B sont identiques et sont choisis parmi les radicaux phényle, 4-méthylphényle, 4-tert.-butylphényle, 4-chlorophényle, 4-bromophényle, biphényl-1-yle(4-phényl-phényle), spécialement ceux dans lesquels A₁ et A₂ sont aussi identiques et choisis parmi les radicaux phényle, 4-méthylphényle, 4-tert.-butylphényle, 4-chlorophényle, 4-bromophényle et biphényl-1-yle.

Des dérivés préférés de pyrrolopyrrole sont ceux dans lesquels A₁ et A₂ désignent chacun un radical 4-tert.-butylphényle, spécialement ceux dans lesquels A et B sont identiques et choisis parmi les radicaux phényle, 4-tert.-butylphényle et 4-méthylphényle, de préférence 4-tert.-butylphényle.

Ainsi, un composé préféré selon l'invention est le dérivé de pyrrolopyrrole dans lequel A, B, A₁ et A₂ désignent chacun un radical 4-tert.-butylphényle.

D'autres dérivés préférés de pyrrolopyrrole sont ceux dans lesquels A₁ et A₂ désignent chacun un radical 4-méthylphényle et A et B sont identiques et choisis parmi les radicaux phényle, 4-tert.-butylphényle et 4-méthylphényle, de préférence 4-tert.-butylphényle.

La préparation des dicétodiarylpyrrolopyrroles de formule (I) est décrite notamment dans le brevet US n° 5 786 487.

De préférence, on utilise les dérivés de 1,4-dicéto-3,6-diphénylpyrrolo[3,4-c]pyrrole présentant la formule (II) suivante : 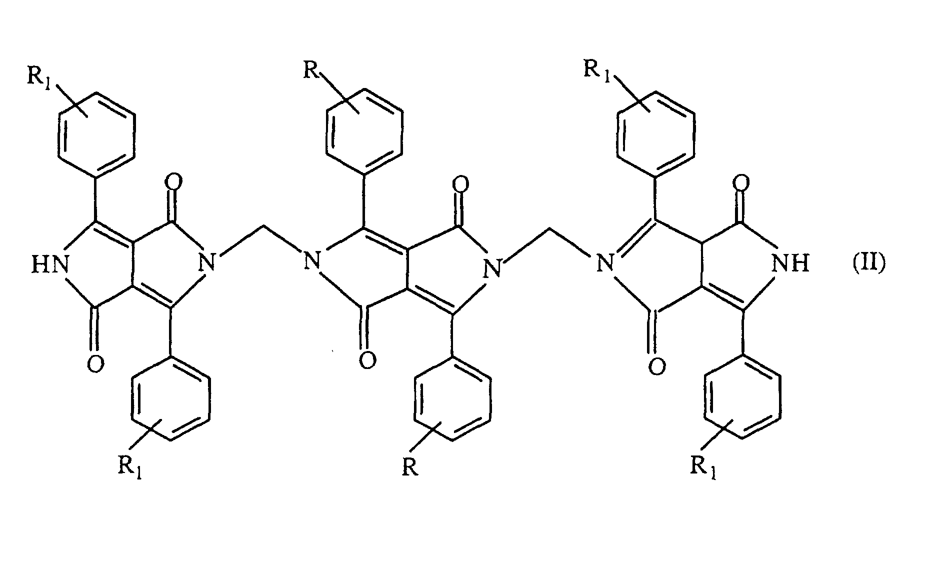 dans laquelle R et R₁ ont les significations indiquées ci-dessous.

| R | R₁ |
|---|---|
| H | H |
| H | Cl |
| H | CH₃ |
| H | tert.-butyle |
| H | 4-phényle |
| CI | H |
| CI | CI |
| CI | CH₃ |
| CI | tert.-butyle |
| CI | 4-phényle |
| CH₃ | H |
| CH₃ | Cl |
| CH₃ | CH₃ |
| CH₃ | tert.-butyle |
| CH₃ | 4-phényle |
| tert.-butyle | H |
| tert.-butyle | CI |
| tert.-butyle | CH₃ |
| tert.-butyle | tert.-butyle |
| tert.-butyle | 4-phényle |
| 4-phényle | H |
| 4-phényle | CI |
| 4-phényle | CH₃ |
| 4-phényle | tert.-butyle |
| 4-phényle | 4-phényle |

Le composé de formule (II) préféré selon l'invention est celui dans lequel R = R₁ = 4-tert.-butyle.

Les dérivés sulfonés des composés ci-dessus peuvent être préparés selon le brevet US 5 786 487, en maintenant tout au long du procédé une température supérieure à la température utilisée pour préparer les dérivés non sulfonés, par exemple supérieure à 40°C si l'on désire un degré de sulfonation élevé ou d'environ 40°C ou inférieure si l'on désire un faible degré de sulfonation, comme par exemple dans le composé de formule (II) contenant 0,5 mole de -SO₃ M.

Les pigments selon l'invention peuvent être avantageusement utilisés dans des compositions de maquillage et des compositions solaires, notamment colorées, destinées à la protection de la peau et/ou des muqueuses telles que les lèvres, sans engendrer de radicaux libres et limitent ainsi la dégradation de la peau et/ou des muqueuses.

Le pigment selon l'invention peut être incorporé dans une composition cosmétique, notamment de maquillage, en une quantité déterminable aisément par l'homme du métier sur la base de ses connaissances générales, et qui peut notamment aller de 0,01 à 50% en poids par rapport au poids total de la composition, de préférence en une quantité allant de 0,5 à 25% en poids. Le pigment peut éventuellement être fixé sur un polymère, notamment greffé, ou encore enrobé.

La composition de l'invention peut se présenter sous forme d'un produit à appliquer sur les lèvres, les yeux, la peau et/ou les fibres kératiniques. Elle contient un milieu cosmétiquement acceptable, c'est-à-dire un milieu compatible avec toutes les matières kératiniques telles que la peau aussi bien du corps humain que du visage, les cheveux, les cils et sourcils. Ce milieu peut se présenter notamment sous forme de suspension, dispersion, solution en milieu solvant, aqueux ou hydroalcoolique, éventuellement épaissie, voire gélifiée; émulsion huile-dans-eau, eau-dans-huile, ou multiple; gel ou mousse; gel émulsionné; dispersion de vésicules, notamment de lipides ioniques ou non; lotion biphase ou multiphase; spray; poudre libre, compactée ou coulée; pâte anhydre.

L'homme du métier pourra choisir la forme appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Lorsque la composition selon l'invention se présente sous forme d'une émulsion, elle peut éventuellement comprendre, en outre, un tensioactif, de préférence en une quantité de 0,01 à 30% en poids par rapport au poids total de la composition.

La composition selon l'invention comprend une phase grasse, notamment constituée de corps gras liquides à température ambiante (25°C en général) et/ou de corps gras solides à température ambiante tels que les cires, les corps gras pâteux, les gommes et leurs mélanges.

Comme corps gras liquides à température ambiante, appelés souvent huiles, utilisables dans l'invention, on peut citer : les huiles hydrocarbonées d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse, notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyl-dodécyle, le diisostéarylmalate, le citrate de triisocétyle, les heptanoates et octanoates d'alcools gras; des esters de polyol comme le dioctanoate de propylèneglycol, le diheptanoate de néopentylglycol, le diisononanoate de diéthylèneglycol et les esters du pentaérythritol; les alcools gras ayant de 12 à 26 atomes de carbone comme l'octyldodécanol, le 2-butyloctanol, le 2-hexyldécanol, le 2-undécylpentadécanol, l'alcool oléique; les huiles fluorées partiellement hydrocarbonées et/ou siliconées; les huiles siliconées comme les polydiméthylsiloxanes (PDMS) volatiles ou non, linéaires ou cycliques, liquides ou pâteuses à température ambiante comme les cyclométhicones, les diméthicones, comportant éventuellement un groupement phényle, comme les phényl triméthicones, les phényltriméthylsiloxydiphénylsiloxanes, les diphénylméthyldiméthyl-trisiloxanes, les diphényldiméthicones, les phényldiméthicones, les polyméthylphénylsiloxanes et leurs mélanges.

Ces huiles peuvent représenter de 0 à 100% en poids par rapport au poids total de la phase grasse.

La composition selon l'invention peut, de plus, comprendre tous les ingrédients classiquement utilisés dans les domaines concernés et plus spécialement dans les domaines cosmétique et dermatologique. Ces ingrédients sont en particulier choisis parmi les conservateurs, les épaississants de phase aqueuse (biopolymères polysaccharidiques, polymères synthétiques) ou grasse, les parfums, les actifs hydrophiles ou lipophiles et leurs mélanges. Les quantités de ces différents ingrédients sont celles classiquement utilisées dans les domaines concernés et sont par exemple de 0,01 à 20% par rapport au poids total de la composition. La nature de ces ingrédients et leur proportion doit être compatible avec l'obtention de compositions selon l'invention, stables, épaissies et brillantes. La composition peut aussi contenir de l'eau à une concentration allant de 0 à 98% du poids total de la composition.

La composition de l'invention peut, en outre, comprendre une phase particulaire additionnelle pouvant être présente à raison de 0 à 35% du poids total de la composition, de préférence de 0,05 à 20%, et qui peut comprendre des pigments et/ou des nacres et/ou des charges utilisés habituellement dans les compositions cosmétiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments autres que le dérivé de DPP peuvent être présents dans la composition à raison de 0 à 25% du poids de la composition finale, et de préférence à raison de 2 à 15%. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone et les laques de baryum, strontium, calcium, aluminium.

Les nacres peuvent être présentes dans la composition à raison de 0 à 20% du poids total de la composition, de préférence à un taux de l'ordre de 1 à 15%. Parmi les nacres utilisables dans l'invention, on peut. citer le mica recouvert d'oxyde de titane, d'oxyde de fer, de pigment naturel ou d'oxychlorure de bismuth tel que le mica titane coloré.

Les charges peuvent être présentes à raison de 0 à 35% du poids total de la composition, de préférence 0,5 à 15%. On peut notamment citer le talc, le stéarate de zinc, le mica, le kaolin, les poudres de Nylon® (Orgasol® notamment) et de polyéthylène, le Téflon®, l'amidon, le nitrure de bore, des microsphères de copolymères telles que l'Expancel® (Nobel Industrie), le polytrap (Dow Corning) et les microbilles de résine de silicone (Tospearl® de Toshiba, par exemple).

Le pigment, objet de l'invention, permet notamment d'augmenter la force colorante des pigments qui lui sont associés et/ou les propriétés goniochromatiques de pigments goniochromatiques comme les pigments multicouches ou à cristaux liquides.

La composition de l'invention peut comprendre, avantageusement une phase grasse solide ou pâteuse, contenant une ou plusieurs gommes et/ou une ou plusieurs cires. Les cires peuvent être hydrocarbonées, fluorées et/ou siliconées et être d'origine végétale, minérale, animale et/ou synthétique. En particulier, les cires présentent une température de fusion supérieure à 25°C et mieux supérieure à 45°C.

Comme cire utilisable dans la composition de l'invention, on peut citer la cire d'abeilles, la cire de Carnauba ou de Candellila, la paraffine, les cires microcristallines, la cérésine ou l'ozokérite; les cires synthétiques comme les cires de polyéthylène ou de Fischer Tropsch, les cires de silicones comme les alkyl ou alkoxy-diméthicones ayant de 16 à 45 atomes de carbone.

Les gommes sont généralement des PDMS à haut poids moléculaire et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0 à 50% en poids de cires, par rapport au poids total de la composition et mieux de 5 à 30%.

La composition selon l'invention peut également comprendre un ou plusieurs solvants organiques cosmétiquement acceptables (tolérance, toxicologie et toucher acceptables). Ces solvants organiques peuvent représenter de 0% à 98% du poids total de la composition et peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des monoalcools inférieurs linéaires ou ramifiés ayant de 1 à 8 atomes de carbone, des polyols, les mono- ou di-alkyl isosorbides dont les groupements alkyle ont de 1 à 5 atomes de carbone, les éthers de glycol, les esters gras.

Cette composition peut avoir l'aspect d'une poudre, crème, pommade, lotion fluide, pâte souple, onguent, solide coulé ou moulé et notamment en stick ou en coupelle.

La composition selon l'invention peut être avantageusement utilisée pour le maquillage de la peau et/ou des lèvres et/ou des fibres kératiniques selon la nature des constituants utilisés. En particulier, la composition de l'invention peut être un bâton de rouge à lèvres ou un brillant à lèvres (gloss en terminologie anglo-saxonne) utilisable tel quel ou pour appliquer sur un film de rouge à lèvres notamment pour en augmenter sa brillance et/ou couleur (top coat en terminologie anglo-saxonne). Elle peut aussi constituer un fond de teint, un produit anti-cernes ou contour des yeux, un eye-liner, un mascara, une ombre à paupières, une poudre, un fard à joues ou un produit de maquillage du corps. Ces compositions peuvent, en outre, contenir des actifs cosmétiques ou dermatologiques, en vue, notamment d'apporter un aspect soin ou traitant à la composition. Ainsi, la composition peut contenir des vitamines et autres actifs lipophiles (lanoline, filtre UVA) ou hydrophiles (hydratants comme la glycérine).

De façon plus spécifique, l'invention a pour objet un produit à lèvres ou un fard à joues (blush).

La composition de l'invention est plus particulièrement sous forme anhydre.

La composition de l'invention peut être obtenue par chauffage des différents constituants à la température de fusion des cires la plus élevée, puis coulage du mélange fondu dans un moule (coupelle ou doigt de gant). Elle peut aussi être obtenue par extrusion comme décrit dans la demande EP-A-667 146.

L'invention a encore pour objet une utilisation cosmétique de la composition ci-dessus pour soigner et/ou maquiller et/ou protéger la peau et/ou les lèvres et/ou les fibres kératiniques ainsi qu'une utilisation de cette composition pour la préparation d'un onguent destiné à traiter et/ou protéger la peau et/ou les lèvres. L'invention a aussi pour objet un procédé de traitement cosmétique de la peau et/ou des lèvres et/ou des fibres kératiniques consistant à appliquer sur la peau et/ou les lèvres et/ou les fibres kératiniques la composition définie ci-dessus.

L'invention a encore pour objet l'utilisation, dans une composition cosmétique colorée, d'un agent de coloration tel que décrit ci-dessus, afin de protéger la peau et/ou les lèvres et/ou les fibres kératiniques contre les méfaits des radicaux libres et/ou lutter contre les signes cutanés du vieillissement, notamment photoinduit. Ces signes du vieillissement sont en particulier les rides, ridules, la peau flasque et/ou jaunie.

L'invention a encore pour objet un procédé de protection cosmétique de la peau et/ou des lèvres et/ou des fibres kératiniques, contre les méfaits des radicaux libres et/ou de lutte contre les signes cutanés du vieillissement photoinduit, consistant à appliquer sur la peau et/ou les lèvres et/ou les fibres kératiniques la composition telle que définie ci-dessus.

Les exemples de compositions ci-après sont donnés à titre illustratif.

### Exemple 1 : Rouge à lèvres.

- Cire de polyéthylène 14 g
- Huile de sésame 78 g
- Pigment dérivé de DPP de formule (II) 5 g
   dans laquelle R = R₁ = 4-tert.-butyle
- Dioxyde de titane 3 g

### Mode de préparation :

- homogénéisation du mélange huile + pigments pendant 45 minutes dans un bain d'huile,
- 3 passages successifs dans un broyeur tricylindre,
- homogénéisation du mélange huile + pigments pendant 30 minutes dans un bain d'huile,
- moulage dans un moule à 42°C et 30 minutes au congélateur.

On obtient un rouge à lèvres d'un orange intense, de couvrance élevée, brillant, stable à la lumière, ne laissant aucune griffe (ou marque) après démaquillage.

### Exemple 2 : Fard à joues (blush).

- Talc 38 g
- Mica 20 g
- Oxychlorure de bismuth 8 g
- Stéarate de zinc 3 g
- Poudre de nylon 20 g
- Pigment dérivé de DPP de formule (II) 5 g
   dans laquelle R = R₁ = 4-tert.-butyle
- Liant gras (*) qsp 100 g
   (*) Mélange d'huiles carbonées contenant :
- 3,6 g de triglycérides d'acide caprique/caprylique,
- 2,0 g d'isoparaffine hydrogénée (non volatile),
- 1,0 g d'huile de jojoba.

### Mode de préparation :

- prémélange de toutes les charges et pigments,
- 5 minutes au Lödige (mélangeur homogénéisateur de poudres),
- addition du liant organique,
- 5 minutes au Lödige,
- passage au jet d'air (CHRISPRO),
- tamisage à 160 microns.

### Exemple 3 : Laque à lèvres.

- Dispersion aqueuse de polymère
   acrylique/styrène 20,0 g matière active
   (Néocryl A-1052 de ZENECA)
- Acétyltributylcitrate 2,5 g
- Vanadate de bismuth 1,5 g
- Dérivé de DPP de formule (II) 1,5 g
   dans laquelle R = R₁ = 4.-tert.-butyle
- Glycérine 1,25 g
- Eau qsp 100 g

### Mode de préparation :

On ajoute à température ambiante l'acétyltributylcitrate, les pigments et la phase aqueuse (glycérine + eau) à la dispersion de polymère, puis on homogénéise.

On obtient une laque à lèvres de couleur orange, stable et couvrante.

## Revendications

1. Composition cosmétique colorée pour application topique, **caractérisée par le fait qu'**elle comprend, dans un milieu cosmétiquement acceptable, une phase grasse et un pigment dérivé de dicétodiarylpyrrolopyrrole, de formule (I) : 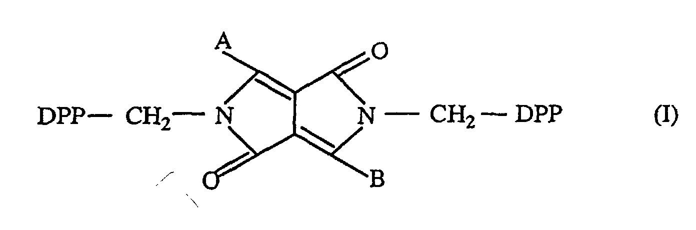 dans laquelle A et B sont des radicaux aryle identiques ou différents et DPP est un radical dicétodiarylpyrrolopyrrolidyle, le dérivé de dicétodiarylpyrrolopyrrole de formule (I) étant substitué par 0 à 6 moles de -SO₃M par mole de dérivé de pyrrolopyrrole, M étant un atome d'hydrogène ou un cation métallique ou ammonium.

2. Composition selon la revendication 1, **caractérisée par le fait que** DPP est un radical 1,4-dicéto-3,6-diarylpyrrolo[3,4-c]pyrrolidyle de formule : 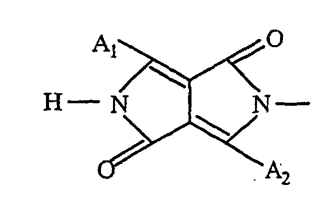 dans laquelle A₁ et A₂ sont des radicaux aryle identiques ou différents.

3. Composition selon la revendication 1 ou 2, **caractérisée par le fait que** A, B, A₁ et A₂ sont des radicaux de formules : 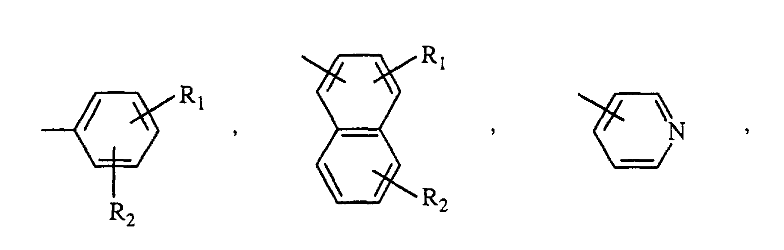 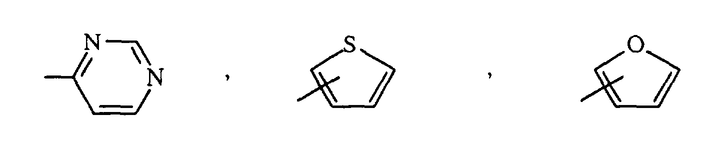 ou 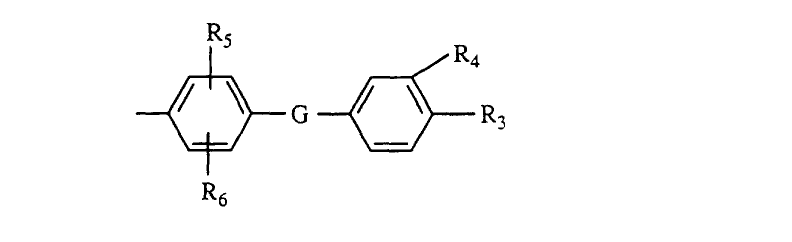 dans lesquelles :
R₁ et R₂ désignent indépendamment l'un de l'autre un atome d'hydrogène ou d'halogène, un radical C₁-C₁₈ alkyle, C₁-C₁₈ alcoxy, C₁-C₁₈ alkylmercapto, C₁-C₁₈ alkylamino, C₁-C₁₈ alcoxycarbonyle, C₁-C₁₈ alkylaminocarbonyle, -CN, -NO₂, trifluorométhyle, C₅-C₆ cycloalkyle, -CH=N-(C₁-C₁₈ alkyle), phényle, 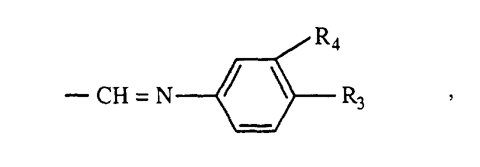 imidazolyle, pyrazolyle, triazolyle, pipérazinyle, pyrrolyle, oxazolyle, benzoxazolyle, benzothiazolyle, benzimidazolyle, morpholinyle, pipéridinyle ou pyrrolidinyle,
G désigne -CH₂-, -CH(CH₃)-, -C(CH₃)₂-, -CH=N-, -N=N-, -O-, -S-, -SO-, -SO₂-, -CONH- ou -NR₇-,
R₃ et R₄ désignent, indépendamment l'un de l'autre, un atome d'hydrogène ou d'halogène, un radical C₁-C₆ alkyle, C₁-C₁₈ alcoxy ou -CN,
R₅ et R₆ désignent, indépendamment l'un de l'autre, un atome d'hydrogène ou d'halogène ou un radical C₁-C₆ alkyle, et
R₇ est un atome d'hydrogène ou un radical C₁-C₆ alkyle.

4. Composition selon la revendication 3, **caractérisée par le fait que** A, B, A₁ et A₂ désignent un groupe de formule : 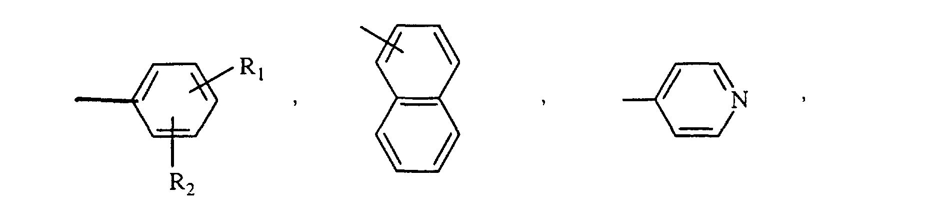 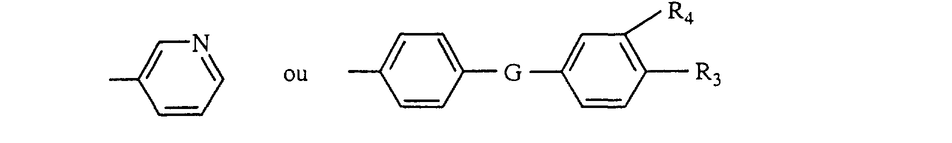 dans laquelle :
R₁ et R₂ désignent chacun, indépendamment l'un de l'autre, un atome d'hydrogène, de chlore ou de brome, un radical C₁-C₄ alkyle, C₁-C₆ alcoxy, C₁-C₆ alkylamino, phényle ou -CN,
G désigne -O-, -NR₇-, -N=N- ou -SO₂-,
R₃ et R₄ désignent un atome d'hydrogène et R₇ est un atome d'hydrogène ou un radical méthyle ou éthyle.

5. Composition selon la revendication 4, **caractérisée par le fait que** A, B, A₁ et A₂ désignent un groupe de formule : 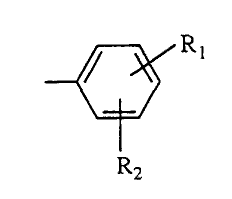 dans laquelle R₁ et R₂ désignent chacun, indépendamment l'un de l'autre, un atome d'hydrogène, de chlore ou de brome, un radical méthyle, tert.-butyle, phényle ou -CN.

6. Composition selon la revendication 5, **caractérisée par le fait que** l'un au moins des radicaux R₁ et R₂ désigne de l'hydrogène.

7. Composition selon la revendication 5 ou 6, **caractérisée par le fait que** l'un au moins des radicaux R₁ et R₂ désigne de l'hydrogène et l'autre est en position 3- ou 4- du noyau phényle.

8. Composition selon l'une quelconque des revendications 5 à 7, **caractérisée par le fait que** A et B sont identiques et sont choisis parmi les radicaux phényle, 4-méthylphényle, 4-tert.-butylphényle, 4-chlorophényle, 4-bromophényle et biphényl-1-yle.

9. Composition selon l'une quelconque des revendications 5 à 8, **caractérisée par le fait que** A₁ et A₂ sont identiques et choisis parmi les radicaux phényle, 4-méthylphényle, 4-tert.-butylphényle, 4-chlorophényle, 4-bromophényle et biphényl-1-yle.

10. Composition selon l'une quelconque des revendications 5 à 9, **caractérisée par le fait que** A₁ et A₂ désignent chacun un radical 4-tert.-butylphényle et A et B sont identiques et choisis parmi les radicaux phényle, 4-tert.-butylphényle et 4-méthylphényle.

11. Composition selon la revendication 10, **caractérisée par le fait que** A₁, A₂, A et B désignent chacun un radical 4-tert.-butylphényle.

12. Composition selon l'une quelconque des revendications 5 à 9, **caractérisée par le fait que** A₁ et A₂ désignent chacun un radical 4-méthylphényle et A et B sont identiques et choisis parmi les radicaux phényle, 4-tert.-butylphényle et 4-méthylphényle.

13. Composition selon la revendication 12, **caractérisée par le fait que** A₁ et A₂ désignent chacun un radical 4-méthylphényle et A et B sont identiques et désignent chacun un radical 4-tert.-butylphényle.

14. Composition selon l'une quelconque des revendications précédentes, **caractérisée par le fait que** le dérivé de pyrrolopyrrole de formule (I) est substitué par 0 à 2 moles de -SO₃M par mole, et de préférence par 0 à 0,75 mole de -SO₃M par mole.

15. Composition selon la revendication 14, **caractérisée par le fait que** le dérivé de pyrrolopyrrole de formule (I) ne contient pas de substituant -SO₃M.

16. Composition selon la revendication 14, **caractérisée par le fait que** le dérivé de pyrrolopyrrole de formule (I) contient 0,5 mole de -SO₃M par mole.

17. Composition selon l'une quelconque des revendications précédentes, **caractérisée par le fait que** le cation métallique M est un cation sodium, potassium ou lithium.

18. Composition selon l'une quelconque des revendications précédentes, **caractérisée par le fait que** le pigment de formule (I) est présent à raison de 0,01 à 50% en poids, par rapport au poids total de la composition.

19. Composition selon la revendication 18, **caractérisée par le fait que** le pigment de formule (I) est présent à raison de 0,5 à 25% en poids, par rapport au poids total de la composition.

20. Composition selon l'une quelconque des revendications précédentes, se présentant sous forme, d'un produit de maquillage de la peau, des lèvres et/ou des fibres kératiniques humaines.

21. Composition selon l'une quelconque des revendications précédentes, se présentant sous forme de mascara, eye-liner, rouge à lèvres, brillant à lèvres, fond de teint, produit anti-cernes ou contour des yeux, fard à joues ou à paupières, poudre, ou produit de maquillage du corps.

22. Composition selon l'une quelconque des revendications précédentes, **caractérisée par le fait que** la phase grasse est choisie parmi les huiles, les cires, les gommes, les corps gras pâteux, et leurs mélanges.

23. Composition selon l'une quelconque des revendications précédentes, **caractérisée en ce qu'**elle contient, en outre, une phase particulaire additionnelle pouvant être présente à raison de 0 à 35% du poids total de la composition.

24. Composition selon l'une quelconque des revendications précédentes, se présentant sous forme anhydre.

25. Utilisation cosmétique de la composition selon l'une des revendications précédentes, pour soigner et/ou maquiller et/ou protéger la peau et/ou les lèvres et/ou les fibres kératiniques.

26. Utilisation de la composition selon l'une des revendications 1 à 24, pour la préparation d'un onguent destiné à traiter et/ou protéger la peau et/ou les lèvres.

27. Utilisation d'un dérivé de dicétodiarylpyrrolopyrrole de formule (I) : 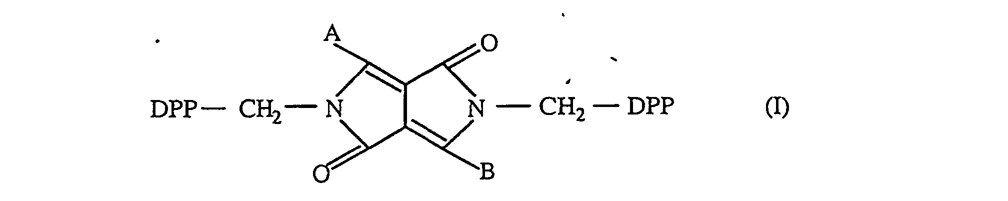 dans laquelle A et B sont des radicaux aryle identiques ou différents et DPP est un radical dicétodiarylpyrrolopyrrolidyle, le dérivé de diaryldicétopyrrolopyrrole de formule (I) étant substitué par 0 à 6 moles de -SO₃M par mole de dérivé de pyrrolopyrrole, M étant un atome d'hydrogène ou un cation métallique ou ammonium en tant que pigment pour la préparation d'une composition cosmétique, destinée à protéger la peau et/ou les lèvres et/ou les phanères contre les méfaits des radicaux libres et/ou lutter contre les signes cutanés du vieillissement.

28. Utilisation d'un dérivé de dicétodiarylpyrrolopyrrole de formule (I) : 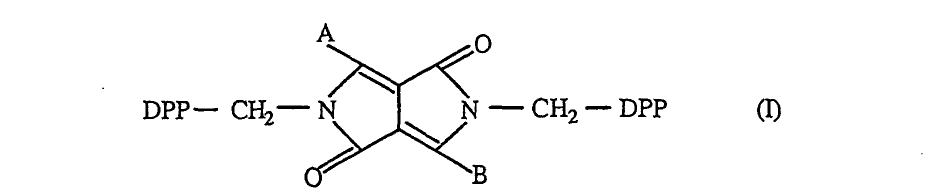 dans laquelle A et B sont des radicaux aryle identiques ou différents et DPP est un radical dicétodiarylpyrrolopyrrolidyle, le dérivé de diaryldicétopyrrolopyrrole de formule (I) étant substitué par 0 à 6 moles de -SO₃M par môle de dérivé de pyrrolopyrrole, M étant un atome d'hydrogène ou un cation métallique ou ammonium en tant que pigment limitant la production de radicaux libres dans une composition cosmétique colorée.

29. Procédé cosmétique pour limiter la production de radicaux libres dans une composition cosmétique colorée appliquée de manière topique sur la peau et/ou les cheveux et/ou les phanères et lutter ainsi contre les signes cutanés du vieillissement, **caractérisé par le fait qu'**il consiste à introduire dans un milieu cosmétiquement acceptable un pigment dérivé de dicétodiarylpyrrolopyrrole de formule (I). 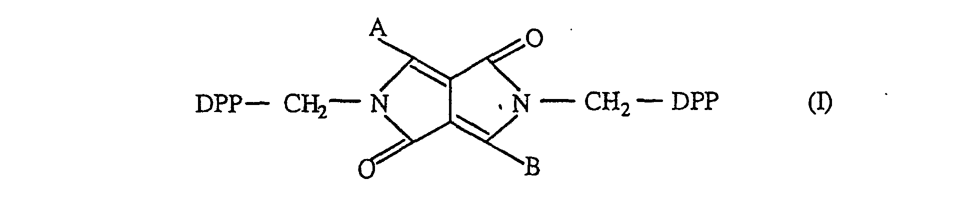 dans laquelle A et B sont des radicaux aryle identiques ou différents et DPP est un radical dicétodiarylpyrrolopyrrolidyle, le dérivé de diaryldicétopyrrolopyrrole de formule (I) étant substitué par 0 à 6 moles de -SO₃M par mole de dérivé de pyrrolopyrrole, M étant un atome d'hydrogène ou un cation métallique ou ammonium, puis à appliquer sur la peau et/ou les cheveux et/ou les phanères, la composition cosmétique ainsi obtenue.

## Patentansprüche

1. Farbige kosmetische Zusammensetzung zur topischen Anwendung, **dadurch gekennzeichnet, dass** sie in einem kosmetisch akzeptablen Medium eine Fettphase und ein von Diketodiarylpyrrolopyrrol abgeleitetes Pigment der Formel (I) enthält: 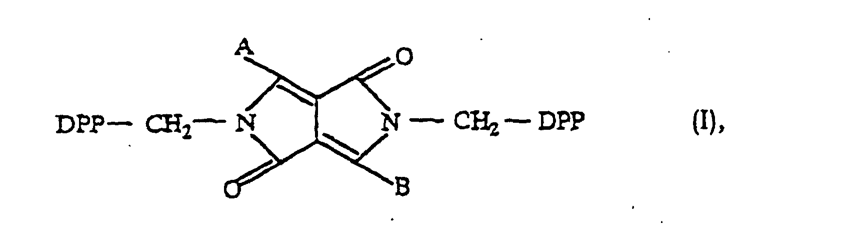 wobei A und B Arylgruppen bedeuten, die gleich oder verschieden sind, und DPP eine Diketodiarylpyrrolopyrrolidylgruppe ist, wobei das Diketodiarylpyrrolopyrrolderivat der Formel (I) mit 0 bis 6 mol -SO₃M pro Mol Pyrrolopyrrolderivat substituiert ist,
worin M ein Wasserstoffatom, ein Metallkation oder Ammonium bedeutet.

2. Zusammensetzung nach Anspruch 1, **dadurch gekennzeichnet, dass** DPP eine 1,4-Diketo-3,6-diarylpyrrolo[3,4-c]pyrrolidylgruppe der folgenden Formel ist: 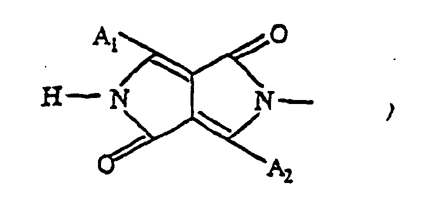 wobei A₁ und A₂ Arylgruppen bedeuten, die identisch oder voneinander verschieden sind.

3. Zusammensetzung nach Anspruch 1 oder 2, **dadurch gekennzeichnet, dass** A, B, A₁ und A₂ Gruppen der folgenden Formeln sind: 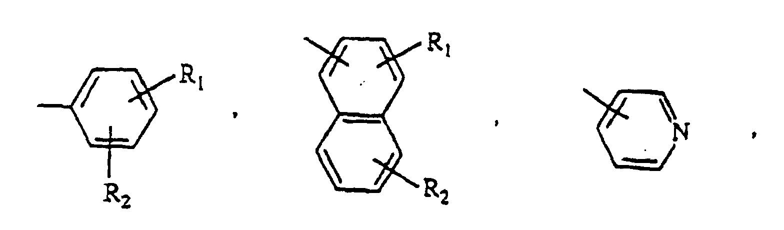 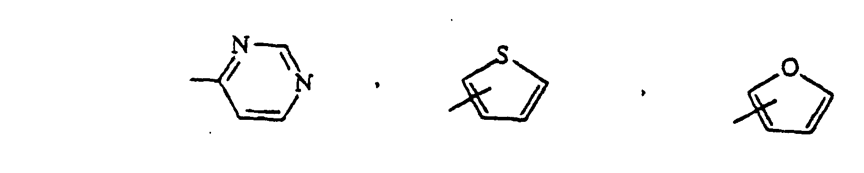 oder 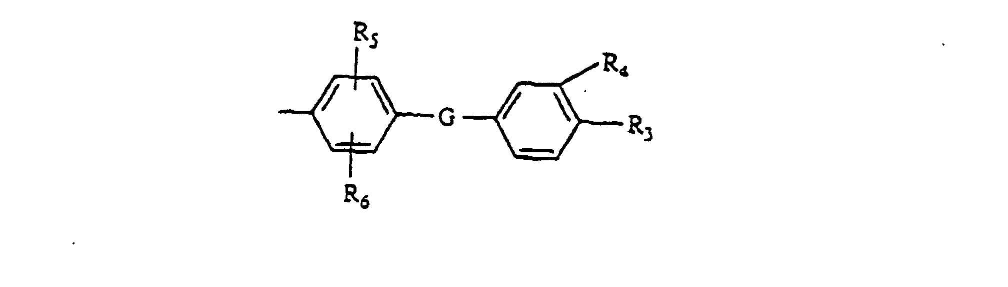 wobei in den Formeln:
- die Gruppen R₁ und R₂ unabhängig voneinander bedeuten: Wasserstoff, Halogen, C₁₋₁₈-Alkyl, C₁₋₁₈-Alkory, C₁₋₁₈-Alkylmercapto, C₁₋₁₈-Alkylamino, C₁₋₁₈-Alkoxycarbonyl, C₁₋₁₈-Alkylaminocarbonyl, -CN, -NO₂, Trifluormethyl, C₅₋₆-Cycloalkyl, -CH=N-(C₁₋₁₈-Alkyl), Phenyl, 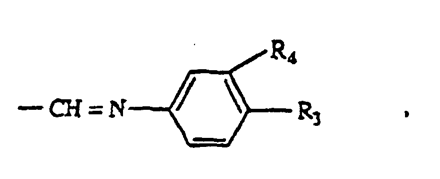 Imidazolyl, Pyrazolyl, Triazolyl, Piperazinyl, Pyrrolyl, Oxazolyl, Benzoxazolyl, Benzothiazolyl, Benzimidazolyl, Morpholinyl, Piperidinyl oder Pyrrolidinyl,
- die Gruppe G bedeutet: -CH₂-, -CH(CH₃)-, -C(CH₃)₂, -CH=N-, -N=N-, -O-, -S-, -SO-, -SO₂-, -CONH- oder -NR₇-,
- die Gruppen R₃ und R₄ unabhängig voneinander bedeuten: Wasserstoff, Halogen, C₁₋₆-Alkyl, C₁₋₁₈-Alkoxy oder -CN,
- die Gruppen R₅ und R₆ unabhängig voneinander bedeuten: Wasserstoff, Halogen oder C₁₋₆-Alkyl, und
- R₇ Wasserstoff oder C₁₋₆-Alkyl bedeutet.

4. Zusammensetzung nach Anspruch 3, **dadurch gekennzeichnet, dass** A, B, A₁ und A₂ eine Gruppe der folgenden Formeln bedeuten: 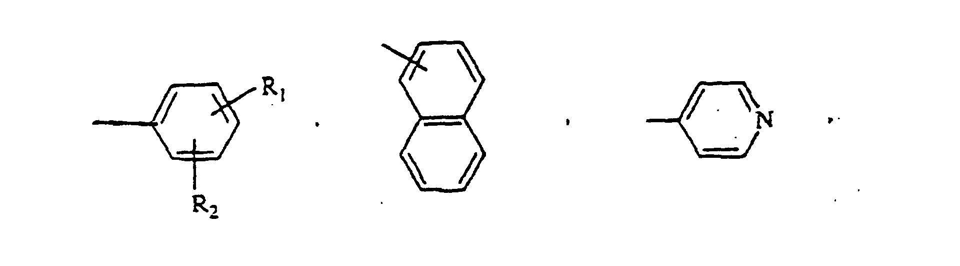 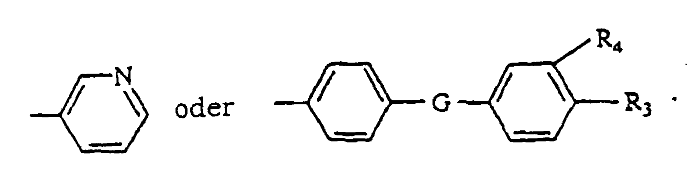 worin bedeuten:
R₁ und R₂ jeweils unabhängig voneinander Wasserstoff, Chlor, Brom, C₁₋₄-Alkyl, C₁₋₆-Alkoxy, C₁₋₆-Alkylamino, Phenyl oder -CN,
G -O-, -NR₇-, -N=N- oder -SO₂-,
R₃ und R₄ Wasserstoff und
R₇ Wasserstoff, Methyl oder Ethyl.

5. Zusammensetzung nach Anspruch 4, **dadurch gekennzeichnet, dass** A, B, A₁ und A₂ eine Gruppe der folgenden Formel bedeuten: 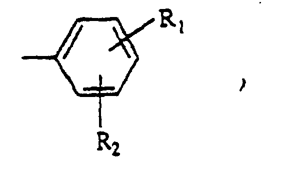 wobei R₁ und R₂ jeweils unabhängig voneinander Wasserstoff, Chlor, Brom, Methyl, *t*-Butyl, Phenyl oder -CN bedeuten.

6. Zusammensetzung nach Anspruch 5, **dadurch gekennzeichnet, dass** mindestens eine der Gruppen R₁ und R₂ Wasserstoff bedeutet.

7. Zusammensetzung nach Anspruch 5 oder 6, **dadurch gekennzeichnet, dass** mindestens eine der Gruppen R₁ und R₂ Wasserstoff bedeutet und die andere Gruppe sich in 3- oder 4-Stellung des Phenylrings befindet.

8. Zusammensetzung nach einem der Ansprüche 5 bis 7, **dadurch gekennzeichnet, dass** A und B identisch sind und unter den Gruppen Phenyl, 4-Methylphenyl, 4-*t*-Butylphenyl, 4-Chlorphenyl, 4-Bromphenyl und Biphenyl-1-yl ausgewählt sind.

9. Zusammensetzung nach einem der Ansprüche 5 bis 8, **dadurch gekennzeichnet, dass** die Gruppen A₁ und A₂ identisch sind und unter den Gruppen Phenyl, 4-Methylphenyl, 4-*t*-Butylphenyl, 4-Chlorphenyl, 4-Bromphenyl und Biphenyl-1-yl ausgewählt sind.

10. Zusammensetzung nach einem der Ansprüche 5 bis 9, **dadurch gekennzeichnet, dass** die Gruppen A₁ und A₂ jeweils eine Gruppe 4-*t*-Butylphenyl bedeuten und die Gruppen A und B identisch sind und unter den Gruppen Phenyl, 4-*t*-Butylphenyl und 4-Methylphenyl ausgewählt sind.

11. Zusammensetzung nach Anspruch 10, **dadurch gekennzeichnet, dass** A₁, A₂, A und B jeweils 4-*t*-Butylphenyl bedeuten.

12. Zusammensetzung nach einem der Ansprüche 5 bis 9, **dadurch gekennzeichnet, dass** die Gruppen A₁ und A₂ 4-Methylphenyl bedeuten und die Gruppen A und B identisch sind und unter den Gruppen Phenyl, 4-*t*-Butylphenyl und 4-Methylphenyl ausgewählt sind.

13. Zusammensetzung nach Anspruch 12, **dadurch gekennzeichnet, dass** die Gruppen A₁ und A₂ jeweils 4-Methylphenyl bedeuten und die Gruppen A und B identisch sind und jeweils 4-*t*-Butylphenyl bedeuten.

14. Zusammensetzung nach einem der vorhergehenden Ansprüche, **dadurch gekennzeichnet, dass** das Pyrrolopyrrolderivat der Formel (I) mit 0 bis 2 mol -SO₃M pro Mol und vorzugsweise 0 bis 0,75 mol -SO₃M pro Mol substituiert ist.

15. Zusammensetzung nach Anspruch 14, **dadurch gekennzeichnet, dass** das Pyrrolopyrrolderivat der Formel (I) keinen Substituenten -SO₃M aufweist.

16. Zusammensetzung nach Anspruch 14, **dadurch gekennzeichnet, dass** das Pyrrolopyrrolderivat der Formel (I) 0,5 mol -SO₃M pro Mol enthält.

17. Zusammensetzung nach einem der vorhergehenden Ansprüche, **dadurch gekennzeichnet, dass** das Metallkation M ein Natrium-, Kalium- oder Lithiumkation ist.

18. Zusammensetzung nach einem der vorhergehenden Ansprüche, **dadurch gekennzeichnet, dass** das Pigment der Formel (I) in einer Menge von 0,01 bis 50 Gew.-%, bezogen auf das Gesamtgewicht der Zusammensetzung, vorliegt.

19. Zusammensetzung nach Anspruch 18, **dadurch gekennzeichnet, dass** das Pigment der Formel (I) in einer Menge von 0,5 bis 25 Gew.-%, bezogen auf das Gesamtgewicht der Zusammensetzung, vorliegt.

20. Zusammensetzung nach einem der vorhergehenden Ansprüche, die als Produkt zum Schminken der menschlichen Haut und/oder der Lippen und/oder der Keratinfasern vorliegt.

21. Zusammensetzung nach einem der vorhergehenden Ansprüche, die als Mascara, Eyeliner, Lippenstift, Lippenglanz, Make-up, Produkt gegen Augenringe, Produkt für die Augenkonturen, Wangenrouge, Lidschatten, Puder oder Produkt zum Schminken des Körpers vorliegt.

22. Zusammensetzung nach einem der vorhergehenden Ansprüche, **dadurch gekennzeichnet, dass** die Fettphase unter den Ölen, Wachsen, Gummis, pastösen Fettsubstanzen und deren Gemischen ausgewählt ist.

23. Zusammensetzung nach einem der vorhergehenden Ansprüche, **dadurch gekennzeichnet, dass** sie ferner eine zusätzliche Partikelphase enthält, die in einer Menge von 0 bis 35 % des Gesamtgewichts der Zusammensetzung vorliegen kann.

24. Zusammensetzung nach einem der vorhergehenden Ansprüche, die wasserfrei ist.

25. Kosmetische Verwendung der Zusammensetzung nach einem der vorhergehenden Ansprüche zur Pflege und/oder zum Schminken und/oder zum Schutz der Haut und/oder der Lippen und/oder der Keratinfasern.

26. Verwendung der Zusammensetzung nach einem der Ansprüche 1 bis 24 zur Herstellung einer Salbe, die zur Behandlung und/oder zum Schutz der Haut und/oder der Lippen vorgesehen ist.

27. Verwendung eines Diketodiarylpyrrolopyrrolderivats der Formel (I): 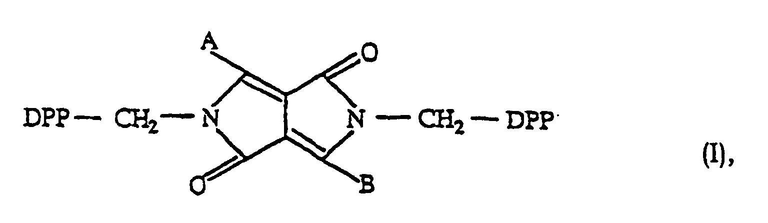 worin die Gruppen A und B Arylgruppen bedeuten, die gleich oder verschieden sind, und DPP eine Diketodiarylpyrrolopyrrolidylgruppe ist, wobei das Diaryldiketopyrrolopyrrolderivat der Formel (I) mit 0 bis 6 mol -SO₃M pro Mol Pyrrolopyrrolderivat substituiert ist, worin M Wasserstoff, ein Metallkation oder Ammonium bedeutet, als Pigment zur Herstellung einer kosmetischen Zusammensetzung, die dazu vorgesehen ist, die Haut und/oder die Lippen und/oder die Hautanhangsgebilde gegen die schädlichen Wirkungen von freien Radikalen zu schützen und/oder die Zeichen der Hautalterung zu bekämpfen.

28. Verwendung eines Diketodiarylpyrrolopyrrolderivats der Formel (I): 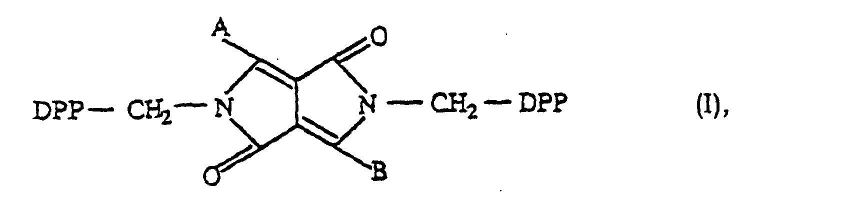 worin A und B Arylgruppen bedeuten, die gleich oder verschieden sind, und DPP eine Diketodiarylpyrrolopyrrolidylgruppe ist, wobei das Diaryldiketopyrrolopyrrolderivat der Formel (I) mit 0 bis 6 mol -SO₃M pro Mol Pyrrolopyrrolderivat substituiert ist, worin M Wasserstoff, ein Metallkation oder Ammonium bedeutet, als Pigment, das die Bildung von freien Radikalen in einer farbigen kosmetischen Zusammensetzung begrenzt.

29. Kosmetisches Verfahren, um die Bildung von freien Radikalen in einer farbigen kosmetischen Zusammensetzung, die topisch auf die Haut und/oder die Haare und/oder die Hautanhangsgebilde aufgetragen wird, zu beschränken und auf diese Weise die Zeichen der Hautalterung zu bekämpfen, **dadurch gekennzeichnet, dass** es darin besteht, in ein kosmetisch akzeptables Medium ein von einem Diketodiarylpyrrolopyrrol der Formel (I) abgeleitetes Pigment einzuarbeiten: 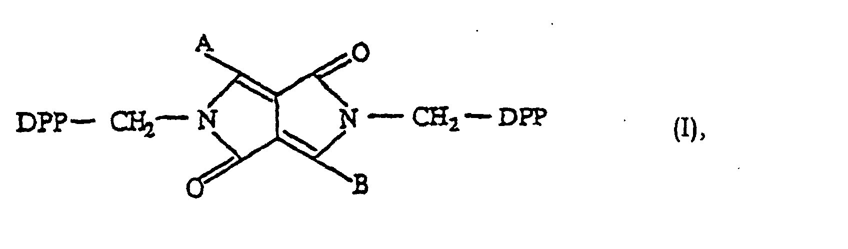 worin die Gruppen A und B Arylgruppen bedeuten, die gleich oder verschieden sind, und DPP eine Diketodiarylpyrrolopyrrolidylgruppe ist, wobei das Diaryldiketopyrrolopyrrolderivat der Formel (I) mit 0 bis 6 mol -SO₃M pro Mol Pyrrolopyrrolderivat substituiert ist, worin M Wasserstoff, ein Metallkation oder Ammonium bedeutet, und anschließend die auf diese Weise erhaltene kosmetische Zusammensetzung auf die Haut und/oder der Haare und/oder die Hautanhangsgebilde aufzutragen.

## Claims

1. Coloured cosmetic composition for topical application, **characterized in that** it comprises, in a cosmetically acceptable medium, a fatty phase and a pigment derived from diketodiarylpyrrolopyrrole of formula (I): 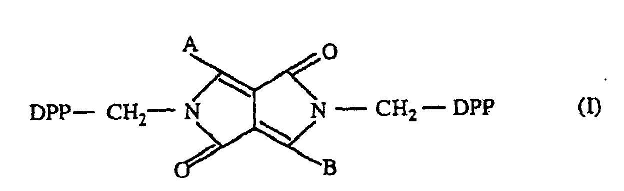 in which A and B are identical or different aryl radicals and DPP is a diketodiarylpyrrolopyrrolidyl radical, the diketodiarylpyrrolopyrrole derivative of formula (I) being substituted by 0 to 6 mol of -SO₃M per mole of pyrrolopyrrole derivative, M being a hydrogen atom or a metal or ammonium cation.

2. Composition according to Claim 1, **characterized in that** DPP is a 1,4-diketo-3,6-diarylpyrrolo[3,4-c]pyrrolidyl radical of formula: 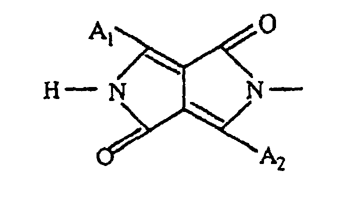 in which A₁ and A₂ are identical or different aryl radicals.

3. Composition according to Claim 1 or 2, **characterized in that** A, B, A₁ and A₂ are radicals of formulae: 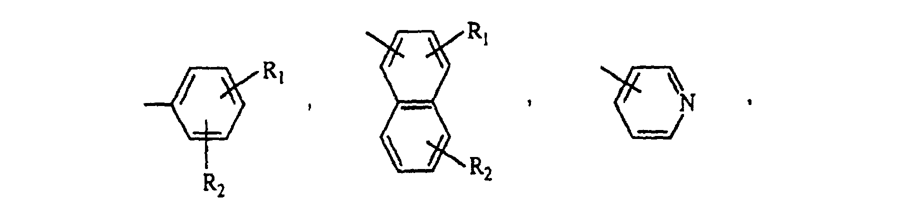 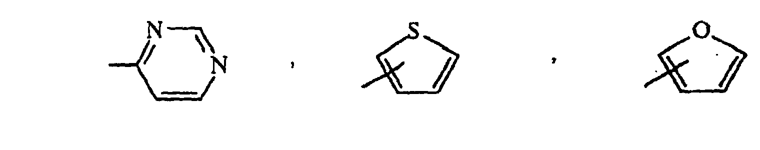 or 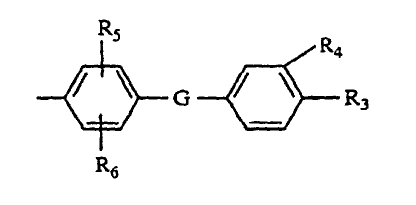 in which:
R₁ and R₂ denote, independently of one another, a hydrogen or halogen atom or a C₁-C₁₈ alkyl, C₁-C₁₈ alkoxy, C₁-C₁₈ alkylmercapto, C₁-C₁₈ alkylamino, C₁-C₁₈ alkoxycarbonyl, C₁-C₁₈ alkylaminocarbonyl, -CN, -NO₂, trifluoromethyl, C₅-C₆ cycloalkyl, -C=N-(C₁-C₁₈ alkyl), phenyl, 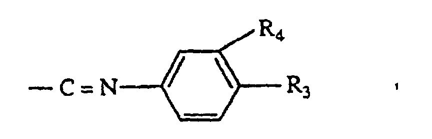 imidazolyl, pyrazolyl, triazolyl, piperazinyl, pyrrolyl, oxazolyl, benzoxazolyl, benzothiazolyl, benzimidazolyl, morpholinyl, piperidinyl or pyrrolidinyl radical,
G denotes -CH₂-, -CH(CH₃)-, -C(CH₃)₂-, -CH=N-, -N=N-, -O-, -S-, -SO-, -SO₂-, -CONH- or -NR₇-,
R₃ and R₄ denote, independently of one another, a hydrogen or halogen atom or a C₁-C₆ alkyl, C₁-C₁₈ alkoxy or -CN radical,
R5 and R₆ denote, independently of one another, a hydrogen or halogen atom or a C₁-C₆ alkyl radical, and
R₇ is a hydrogen atom or a C₁-C₆ alkyl radical.

4. Composition according to Claim 3, **characterized in that** A, B, A₁ and A₂ denote a group of formula: 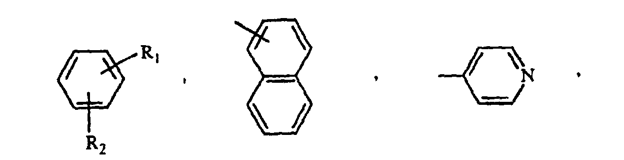 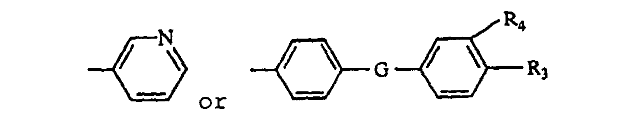 in which
R₁ and R₂ each denote, independently of one another, a hydrogen, chlorine or bromine atom or a C₁-C₄ alkyl, C₁-C₆ alkoxy, C₁-C₆ alkylamino, phenyl or -CN radical,
G denotes -O-, -NR₇-, -N=N- or -SO₂-,
R₃ and R₄ denote a hydrogen atom, and
R₇ is a hydrogen atom or a methyl or ethyl radical.

5. Composition according to Claim 4, **characterized in that** A, B, A₁ and A₂ denote a group of formula: 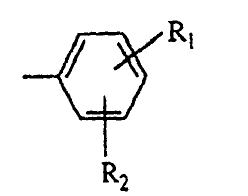 in which R₁ and R₂ each denote, independently of one another, a hydrogen, chlorine or bromine atom or a methyl, tert-butyl, phenyl or -CN radical.

6. Composition according to Claim 5, **characterized in that** at least one of the R₁ and R₂ radicals denotes hydrogen.

7. Composition according to Claim 5 or 6, **characterized in that** at least one of the R₁ and R₂ radicals denotes hydrogen and the other is in the 3- or 4-position of the phenyl nucleus.

8. Composition according to any one of Claims 5 to 7, **characterized in that** A and B are identical and are chosen from the phenyl, 4-methylphenyl, 4-tert-butylphenyl, 4-chlorophenyl, 4-bromophenyl and biphenyl-1-yl radicals.

9. Composition according to any one of Claims 5 to 8, **characterized in that** A₁ and A₂ are identical and are chosen from the phenyl, 4-methylphenyl, 4-tert-butylphenyl, 4-chlorophenyl, 4-bromophenyl and biphenyl-1-yl radicals.

10. Composition according to any one of Claims 5 to 9, **characterized in that** A₁ and A₂ each denote a 4-tert-butylphenyl radical and A and B are identical and are chosen from the phenyl, 4-tert-butylphenyl and 4-methylphenyl radicals.

11. Composition according to Claim 10, **characterized in that** A₁, A₂, A and B each denote a 4-tert-butylphenyl radical.

12. Composition according to any one of Claims 5 to 9, **characterized in that** A₁ and A₂ each denote a 4-methylphenyl radical and A and B are identical and are chosen from the phenyl, 4-tert-butylphenyl and 4-methylphenyl radicals.

13. Composition according to Claim 12, **characterized in that** A₁ and A₂ each denote a 4-methylphenyl radical and A and B are identical and each denote a 4-tert-butylphenyl radical.

14. Composition according to any one of the preceding claims, **characterized in that** the pyrrolopyrrole derivative of formula (I) is substituted by 0 to 2 mol of -SO₃M per mole and preferably by 0 to 0.75 mol of -SO₃M per mole.

15. Composition according to Claim 14, **characterized in that** the pyrrolopyrrole derivative of formula (I) does not comprise an -SO₃M substituent.

16. Composition according to Claim 14, **characterized in that** the pyrrolopyrrole derivative of formula (I) comprises 0.5 mol of -SO₃M per mole.

17. Composition according to any one of the preceding claims, **characterized in that** the metal cation M is a sodium, potassium or lithium cation.

18. Composition according to any one of the preceding claims, **characterized in that** the pigment of formula (I) is present in a proportion of 0.01 to 50% by weight with respect to the total weight of the composition.

19. Composition according to Claim 18, **characterized in that** the pigment of formula (I) is present in a proportion of 0.5 to 25% by weight with respect to the total weight of the composition.

20. Composition according to any one of the preceding claims, which is provided in the form of a product for making up human skin, lips and/or keratinous fibres.

21. Composition according to any one of the preceding claims, which is provided in the form of a mascara, eyeliner, lipstick, lip gloss, foundation, concealer or eye contour product, blusher, eyeshadow, powder or make-up for the body.

22. Composition according to any one of the preceding claims, **characterized in that** the fatty phase is chosen from oils, waxes, gums, pasty fatty substances and their mixtures.

23. Composition according to any one of the preceding claims, **characterized in that** it furthermore comprises an additional particulate phase which can be present in a proportion of 0 to 35% of the total weight of the composition.

24. Composition according to any one of the preceding claims, which is provided in anhydrous form.

25. Cosmetic use of the composition according to one of the preceding claims for caring for and/or making up and/or protecting the skin and/or lips and/or keratinous fibres.

26. Use of the composition according to one of Claims 1 to 24 for preparing a salve intended to treat and/or protect the skin and/or lips.

27. Use of a diketodiarylpyrrolopyrrole derivative of formula (I) 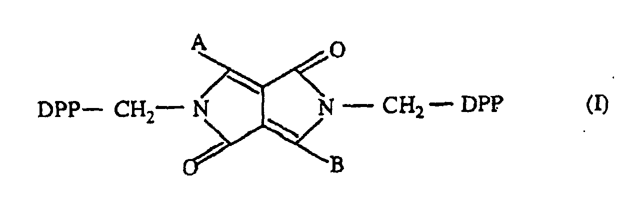 in which A and B are identical or different aryl radicals and DPP is a diketodiarylpyrrolopyrrolidyl radical, the diketodiarylpyrrolopyrrole derivative of formula (I) being substituted by 0 to 6 mol of -SO₃M per mole of pyrrolopyrrole derivative, M being a hydrogen atom or a metal or ammonium cation, as pigment in the preparation of a cosmetic composition intended to protect the skin and/or lips and/or keratinous fibres against the damaging effects of free radicals and/or to combat cutaneous signs of ageing.

28. Use of a diketodiarylpyrrolopyrrole derivative of formula (I) 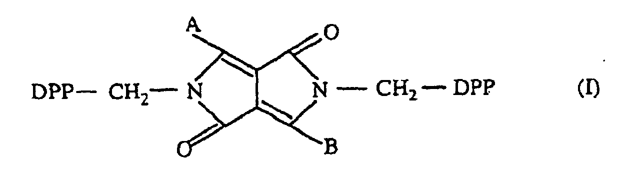 in which A and B are identical or different aryl radicals and DPP is a diketodiarylpyrrolopyrrolidyl radical, the diketodiarylpyrrolopyrrole derivative of formula (I) being substituted by 0 to 6 mol of -SO₃M per mole of pyrrolopyrrole derivative, M being a hydrogen atom or a metal or ammonium cation, as pigment which limits production of free radicals in a coloured cosmetic composition.

29. Cosmetic process for limiting the production of free radicals in a coloured cosmetic composition applied topically to the skin and/or lips and/or keratinous fibres and thus combating cutaneous signs of ageing, **characterized in that** it consists of introducing, into a cosmetically acceptable medium, a pigment derived from diketodiarylpyrrolopyrrole of formula (I) 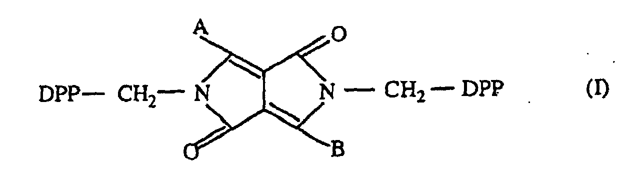 in which A and B are identical or different aryl radicals and DPP is a diketodiarylpyrrolopyrrolidyl radical, the diketodiarylpyrrolopyrrole derivative of formula (I) being substituted by 0 to 6 mol of -SO₃M per mole of pyrrolopyrrole derivative, M being a hydrogen atom or a metal or ammonium cation, and of then applying the cosmetic composition thus obtained to the skin and/or lips and/or keratinous fibres.
